# EUROPÄISCHE PATENTSCHRIFT

(11) **EP 0 765 864 B1**
(45) Veröffentlichungstag und Bekanntmachung des Hinweises auf die Patenterteilung: **26.05.1999**
(21) Anmeldenummer: 96111143.2
(22) Anmeldetag: 11.07.1996
(51) Int. Cl.: C07C 227/40, C07C 303/44

(54) **Verfahren zur Abtrennung von Aminosäuren und Aminosulfonsäuren durch Adsorption an Zeolithen**
Process for the separation of amino acids and aminosulfonic acids by adsorption to zeolites
Procédé pour la séparation des aminoacides et acides aminosulfoniques par adsorption dans zéolithes

(30) Priorität: 26.09.1995 DE 19535751
(43) Veröffentlichungstag der Anmeldung: 02.04.1997
(73) Patentinhaber: Degussa Aktiengesellschaft, 60311 Frankfurt (DE)
(72) Erfinder: Stockhammer, Stefan, 63579 Freigericht (DE); Schäfer-Treffenfeldt, Wiltrud, Prof., 63179 Obertshausen (DE); Knaup, Günther, Dr., 63486 Bruchköbel (DE); Drauz, Karlheinz, Prof., 63579 Freigericht (DE); Sextl, Elfriede, Dr., 63826 Geiselbach (DE)

(56) Entgegenhaltungen:
- EP-A- 0 571 742
- EP-A- 0 645 371
- WO-A-94/00213
- US-A- 4 910 336

## Beschreibung

Die Erfindung betrifft ein Verfahren zur Abtrennung von Aminosäuren aus vorzugsweise wäßrigen Lösungen, die diese als Verunreinigungen enthalten durch Adsorption der Aminosäuren an Zeolithen.
Lösungen bei denen das erfindungsgemäße Verfahren zur Anwendung kommen kann, resultieren z. B. aus der technischen Synthese von Oligopeptiden, bei der immer zum Teil nicht unerhebliche Restkonzentrationen der als Ausgangsstoffe dienenden Aminosäuren, zusammen mit dem erwünschten Endprodukt in Lösung verbleiben. Ein entscheidender Schritt bei der Produktion reiner Peptide besteht in der Abtrennung der Ausgangsprodukte. Besonders schwierig ist die Abtrennung der Aminosäuren von den Dipeptiden, wenn zur Herstellung Verfahren benutzt werden, bei denen die für den Schutz der Aminogruppe erforderliche Schutzgruppe nach der Kopplung direkt abgespalten wird (z. B. bei der Peptidkopplung mit N-Carbonsäureanhydriden) und zur Kopplung eine zweite ungeschützte Aminosäure eingesetzt wird. Die in diesen Fällen in Lösung vorliegenden freien Aminosäuren und freien Peptide haben oft sehr ähnliche PKI-Werte und daher ein sehr ähnliches Lösungsverhalten. Eine Reinigung durch Kristallisation ist daher oft nicht oder nur unter großen Verlusten möglich.
Für diese Reinigung finden nach dem heutigen Stand der Technik verschiedene chromatographische Verfahren, wie sie in (1) und (2) zusammenfassend beschrieben werden, Anwendung.
Bei der Verteilungschromatographie nutzt man die unterschiedlichen Verteilungsgleichgewichte von Aminosäuren und Peptiden zwischen zwei unterschiedlichen Lösungsmittelsystemen (wäßrig und organisch). Liegen diese Verteilungsgleichgewichte nicht ausreichend weit auseinander, wird eine

Reinigung nach dieser Methode schwierig bis unmöglich. Die Affinitätschromatographie, bei der die Unterschiede in den Bindungsstärken zu speziellen Reaktionspartnern ausgenutzt werden, ist nur für sehr kleine Mengen geeignet. Chromatographieverfahren, die auf hydrophoben Wechselwirkungen zwischen einem Träger und den zu reinigenden Stoffen beruhen, (wie sie auch für Peptide und Proteine in der PCT (SE 93/00582) beschrieben sind) machen sich die Abhängigkeit dieser Wechselwirkungen und damit der Bindung an dem Trägermaterial von der Salzkonzentration im Elutionsmedium zunutze. Bei diesen Verfahren ist oft auch die Zugabe unpolarer, organischer Substanzen für eine ausreichende Trennleistung notwendig.

Im Vergleich zu den bisher genannten Verfahren hat die Ionenaustausch-Chromatographie die größere Bedeutung in technischen Prozessen erlangt. Die Trennung von Aminosäuren und Peptiden beruht hier auf den Unterschieden der isoelektrischen Punkte der Substanzen. Aminosäuren und Peptide werden bei saurem pH-Wert in ihrer Kationenform durch Ionentausch an einen Kationentauscher gebunden. Die Trennung erfolgt durch Elution mit einem steigenden pH-Gradienten des Elutionsmittels. Bei dem pH-Wert, der ihrem eigenen isoelektrischen Punkt entspricht, werden die einzelnen Aminosäuren und Peptide freigesetzt und eluiert.

Alle genannten chromatographischen Verfahren haben den Nachteil, daß für Substanzen mit ähnlichen und vergleichbaren funktionellen Gruppen mehrere Stufen zur Erzielung einer ausreichenden Reinheit erforderlich sind. Daraus resultiert die Notwendigkeit mehrer Reinigungszyclen, in denen größere Wertstoffverluste nicht vermeidbar sind. Zusätzlich wird durch Elution unter Zugabe von Salzen oder durch pH-Shift die Salzfracht in den Lösungen zum Teil erheblich erhöht. Diese Salze und eventuell auch andere notwendige Zusatzstoff müssen dann mit erheblichem Aufwand wieder entfernt werden.

Ein weiteres Verfahren, bei dem die Trennung und Reinigung durch Unterschiede im Molekulargewicht und den Abmessungen der zu trennenden Moleküle erfolgt, ist die Gelfiltration. Dabei wird eine poröse Matrix, meist auf organischer Basis, eingesetzt. Größere Moleküle können nicht in die Poren diffundieren und werden schnell eluiert, wohingegen kleinere Moleküle zurückgehalten werden. Von Nachteil ist dabei aber der für einen ausreichenden Durchsatz aufzubringende hohe Druck.

Aus der EP 0 571 742 A2 ist ein Verfahren zur Abtrennung von Aminosäuren aus Fermentationsbrühen durch Adsorption dieser Verbindungen an Zeolithe bekannt. Diese enthalten Polypeptide, die jedoch in Hinblick auf die abzutrennenden Aminosäuren unspezifisch zusammengesetzt sind.

Aufgabe der Erfindung ist es, ein Verfahren zur Verfügung zu stellen, das eine effektive Abtrennung der nach der Herstellung der Di- oder Oligopeptide in der Lösung verbliebenen, nicht umgesetzten Aminosäuren ermöglicht.

Gegenstand der Erfindung ist ein Verfahren zur Trennung von in Lösung vorliegenden Aminosäuren und/oder Aminosulfonsäuren von aus ihnen hergestellten Dipeptide und/oder Oligopeptide, das dadurch gekennzeichnet ist, daß man die Lösungen bei einem geeigneten pH-Wert mit einem Zeolith, vorzugsweise der Typen TA, FAU, MOR oder MSI gegebenenfalls in mehreren Schritten in Kontakt bringt,und die in Lösung verbleibenden Produkte von den am Zeolith adsorbierten Aminosäuren und/oder Aminosulfonsäuren abtrennt.
Die Adsorptionstemperatur kann dabei zwischen den Schmelzund Siedepunkten des verwendeten Lösungsmittels, vorzugsweise zwischen 15 und 35 °C liegen.
Bei den abzutrennenden Aminosäuren kann es sich um alle organischen Verbindungen handeln, die jeweils mindestens eine Aminogruppe sowie eine Carbonsäure- oder eine Sulfonsäuregruppe enthalten. Bevorzugt sind Aminosäuren und/oder Aminosulfonsäuren, bei denen die Amino- und Säuregruppe über einenm C₁ -C₄ -Alkylenrest verbunden sind.

Falls die Aminosäuren oder Aminosulfonsäuren ein chirales Zentrum enthalten, ist das Verfahren auf beide Enantiomere anwendbar. Bei den Aminogruppen kann es sich um primäre, sekundäre oder teriäre Amingruppen handeln.
Die mit dem erfindungemäßen Verfahren abtrennbaren Aminosäuren und Aminosulfonsäuren können noch weitere, funktionelle Gruppen, wie z. B.
Carboxyl, Sulfenyl, Hydroxyl, Amino- Thionyl, Guanidin; Heteroaryl tragen. Diese Gruppen können gegebenenfalls auch mit in der Peptidchemie gebräuchlichen Schutzgruppen, wie z. B. Benzyloxycarboxyl-, t-Butoxycarboxyl, Trifluoracetyl-, Tosyl-für Aminogruppen bzw. Guanidinogruppen oder Alkylester für Carboxylgruppen tragen.
Das Verfahren läßt sich besonders vorteilhaft zur Abtrennung von Aminosäuren und/oder Aminosulfonsäuren von diese enthaltende oder aus ihnen hergestellten Produkten anwenden, wenn die Produkte ebenfalls freie Amino- und Carbonsäure- bzw. Sulfonsäuregruppen enthalten. Bevorzugt wird das Verfahren zur Abtrennung von nichtumgesetzten Aminosäuren und/oder Aminosulfonsäuren von Produkten eingesetzt, die durch die Verknüfung von zwei oder mehr Aminosäuren hergestellt werden. Besonders bevorzugt sind Verbindungen, bei denen die Verknüpfung über Amidbindungen erfolgt.

Bevorzugt wird das erfindungsgemäße Verfahren zur Abtrennung von Di- und/oder Tripeptiden aus überwiegend wäßrigen Lösungen angewendet, in denen die die Peptide bildenden Aminosäuren, bzw. Aminosulfonsäuren sämtlich oder zum Teil enthalten sind.

Beispielhaft angeführt seien die vorzugsweise durchgeführten Trennungen von Dipeptiden und den in ihnen enthaltenen α-L-Aminosäuren:

Ala + Pro von Ala-Pro
Val + Pro von Val-Pro
Gly + Gln von Gly-Gln
Tyr + Arg von Tyr-Arg
Gly + Glu von Gly-Glu

Als ein Beispiel zur Abtrennung von β-L-Aminosäuren ist anzusehen:

β-Ala + His von β-Ala-His

Dasselbe gilt für die Abtrennung von α-L-Aminosäuren aus Tripeptide enthaltenden Lösungen:

Gly + Tyr von Gly-Gly-Tyr.

Als ein Beispiel für die Abtennung einer Diaminocarbonsäure, bei der eine Aminogruppe durch eine Trifluoracetylgruppe geschützt ist, ist zu nennen:

ε-TFA - Lys + Pro von ε-TFA-Lys-Pro
(TFA: Trifluoracetyl)

Die praxisnahe Prüfung des erfindungsgemäßen Verfahrens zeigt die universelle Anwendbarkeit, obwohl Aminosäuren eine Vielzahl von unterschiedlichen chemischen Eigenschaften besitzen können.

Entsprechend weisen die aus proteinogenen Aminosäuren bestehenden Peptide
a) "hydrophobe" Seitenketten: *Gly*, *Ala, Pro, Val* oder/und
b) "polare" Seitenketten: *Tyr, Gln* oder/und
c) "saure" Seitenketten: *Glu* oder/und
d) "basische" Seitenketten: *Arg, Lys*
auf, wie anhand der aufgeführten Aminosäuren beispielhaft belegt wird.

Erfindungsgemäß gelingt auch die Abtrennung von Aminosäuren mit sekundären Amino-Gruppen, wie z. B.

Sar + Ala von Sar-Ala (Sar = Sarkosin).

Dasselbe gilt auch für die Abtrennung von Aminosulfonsäuren,wie z. B.

Ala + Tau von Ala-Tau (Tau = Taurin)

Da die Adsorption von Aminosäuren im gesamten pH-Bereich zwischen 1 und dem jeweiligen JP möglich ist, wird in Lösungen mit dem entsprechenden pH-Wert keine pH-Korrektur notwendig.

Vorzugsweise führt man die Adsorption bei einem pH <JP (Isoelektrischer Punkt) durch, während die Desorption vorzugsweise bei einem pH >JP vorgenommen wird.
Liegen die Isoelektrischen Punkte zweier aus der Lösung zu entfernender Aminosäuren bzw. Aminosulfonsäuren deutlich auseinander, wird der Adsorptionsschritt gegebenenfalls bei unterschiedlichen pH-Werten wiederholt.

Als Zeolithe werden bevorzugt solche der Typen FAU, MSI oder Mordenit eingesetzt,die einen Modul von 15 bis 200 besitzen.
Die Zeolithe als solche sind aus dem Stand der Technik bekannt.

Die zu reinigende Lösung wird beispielsweise mit dem Zeolithen durch direkte Zugabe von Zeolithpulver oder -formkörper zur Lösung in einem durchmischten Behälter in Kontakt gebracht. Das Abtrennen der gereinigten Wertstofflösung vom Zeolithpulver oder den Formkörpern erfolgt durch anschließende Filtration.

Eine weitere Möglichkeit der technischen Umsetzung besteht darin, die zu reinigende Lösung kontinuierlich durch eine mit Zeolithpulver oder Formkörpern gefüllte Kolonne zu

fördern, wobei Formkörper aufgrund der geringen Druckverluste bevorzugt werden. Die freien Aminosäuren werden dabei in der Kolonne am Zeolithen adsorbiert, während die Dipeptide nahezu vollständig im Ablauf enthalten sind. Der mit Aminosäuren beladene Zeolith kann, wenn die abgetrennten Aminosäuren nicht zurückgewonnen werden sollen, durch Erhitzen bei Temperaturen zwischen 400 und 900 °C, z. B. in einem Drehrohrofen, regeneriert werden. Die adsorbierten Aminosäuren können aber auch in wäßrigen Lösungen bei pH-Werten zwischen 10 und 12 vollständig desorbiert und dadurch wiedergewonnen werden. Der Zeolith kann danach erneut zur Reinigung von Peptiden eingesetzt werden.

Aufgrund der günstigen Lage des Adsorptionsgleichgewichtes gelingt es, die Aminosäuren bzw. Aminosulfonsäuren, bei Zugabe einer entsprechenden Zeolithmenge, in einem Schritt nahezu vollständig aus der Lösung zu entfernen. Selbst wenn in manchen Fällen dabei auch der Wertstoff in geringen Mengen adsorbiert wird, bleibt immer eine deutliche Trennleistung zwischen freien Aminosäuren und Wertstoff zu beobachten.

Im Vergleich zum Stand der Technik zeichnet dies -unter anderem- das erfindungsgemäße Verfahren aus.
Dazu kommt die hohe Effizienz, da bei der Abtrennung geringer Mengen an Nebenprodukten bzw. Ausgangsverbindungen aus Reaktionslösungen, wie sie bevorzugt eingesetzt werden, von den zu reinigenden Wertstoffen im Vergleich zur Ionenaustauscher-Chromatographie keine Kapazität des Adsorptionsmittels durch den Wertstoff (hier Peptide) beansprucht wird.
Die benötigte Adsorbensmenge richtet sich allein nach der Menge der zu entfernenden Substanzen.

Im Gegensatz zur Gelfiltration erfolgt die Trennung ohne die Anwendung hoher Drücke, die dort wegen der geringen Beladungsdichte/ - kapazität notwendig werden.
Als vorteilhaft erweist sich auch, daß erfindungsgemäß kein Aufsalzen und auch keine Verdünnung der Wertstofflösung notwendig ist.

Die Konzentration der gewünschten Produkte erstreckt sich in Abhängigkeit von ihrer Löslichkeit und den nach dem jeweils gewählten Verfahren erzielbaren
Konzentrationswerten im allgemeinen auf einen Bereich von 1 Gew.-% bis 60 Gew.-%, bezogen auf die sie enthaltende Lösung, insbesondere 4 bis 30 Gew.-%.

Die Konzentration der abzutrennenden, z. B. als Ausgangsverbindungen dienenden Aminosäuren beläuft sich im allgemeinen auf eine nach der Umsetzung aufzufindende Restkonzentration von 0,01 g/l bis auf den Wert, der durch die Löslichkeitsgrenze der jeweils eingesetzten Ausgangsverbindung vorgegeben ist.
Drückt man das Verhältnis der einzelnen abzutrennenden Verbindung, wie z. B. der oder den Aminosäure(n) zu dem zu reinigenden Produkt in g/l aus, findet man einen Bereich von 1 : 1000 bis 1 : 1,5 insbesondere 1 : 300 bis 1 : 1,5, in dem das erfindungsgemäße Verfahren erfolgreich anzuwenden ist.

Die Charakterisierung der verwendeten Zeolithe entspricht der Einordnung nach W.M. Meier, D.H. Olson "Atlas of Zeolite Structure Types, 2nd Ed. Butterworth-Heinemann, London, 1987.

Das gilt insbesondere für

| | |
|---|---|
| Zeolith A | ^ Zeolith TA |
| Zeolith DAY | ^ Zeolith FAU |
| Mordenit | ^ MOR |
| ZSM 5 | ^ MSI |

Die zur Kennzeichnung des ZSM 5 Typs durch Bindestrich verbundene Zahl in den Beispielen entspricht dem jeweiligen SiO₂/Al₂O₃-Verhältnis.

Literatur:
1. Ullmann's Enzyklopädie, Vol A 19, pp 168, (1991)
2. J.P. Greenstein, M. Winitiz, ,,Chemistry of the Amino Acids" J. Wiley , New York, 1961, Vol. 2, pp 1366-1511.

### Beispiele

Die angefügten Beispiele belegen die Durchführbarkeit des erfindungsgemäßen Verfahrens.

In Laborversuchen wurden jeweils 30 ml einer Lösung, die verschiedene Konzentrationen an Peptiden und freien Aminosäuren enthielt, mit je 3 g Zeolithpulver versetzt und bis zur sicheren Einstellung des Adsorptionsgleichgewichtes mehrere Stunden geschüttelt.
Nach der Adsorption wurde das Zeolithpulver über eine Membran abfiltriert und der Überstand analysiert.

Die untersuchten Substanzen sind zusammen mit den Analyseergebnissen und der erzielten prozentualen Abreicherung durch den beschriebenen Adsorptionsschritt in Tabelle 1 aufgeführt.

| **Abtrennung freier Aminosäuren von Dipeptiden oder verwandten Substanzen** | | | |
|---|---|---|---|
| Substanz | Konzentration in der Ausgangslösung ( g / l ) | Konzentration in der Lösung nach Kontakt mit dem Zeolithen ( g / l ) | Prozentuale Abreicherung |
| ***Zeolith*** | | | |
| Ala-Pro | 285,9 | 286,5 | 0 |
| Ala | 4,0 | 0,6 | 85,0 |
| Pro | 2,4 | 0,0 | 100 |
| ***ZSM5/28*** | | | |
| Val-Pro | 324,9 | 326,1 | 0 |
| Val | 14,1 | 2,1 | 85,1 |
| Pro | 4,5 | 0,8 | 82,2 |
| ***ZSM5/28*** | | | |
| Gly-Gln | 89,1 | 82,0 | 7,9 |
| Gly | 5,2 | 0,2 | 96,2 |
| Gln | 2,0 | 0,2 | 90,0 |
| ***ZSM5/28*** | | | |
| TFA-Lys-Pro | 39,1 | 39,9 | 0 |
| TFA-Lys | 3,0 | 1,0 | 66,6. |
| Pro | 2,8 | 0,1 | 96,4 |
| ***ZSM5/45*** | | | |
| Tyr-Arg | 38,4 | 17,2 | 55,2 |
| Tyr | 0,5 | 0,3 | 40,0 |
| Arg | 4,3 | 1,2 | 72,1 |
| ***DAY 15*** | | | |
| Gly-Gly-Tyr | 5,8 | 5,45 | 6,0 |
| Gly-Tyr | 3,25 | 3,1 | 4,6 |
| Gly | 5,75 | 1,5 | 73,9 |
| Tyr | 0,3 | 0,1 | 66,6 |
| ***ZSM5/28*** | | | |
| Gly-Glu | 8,25 | 7,8 | 5,4 |
| Gly | 9,6 | 6,1 | 36,4 |
| Glu | 5,0 | 2,4 | 52,0 |
| ***ZSM5/28*** | | | |

## Patentansprüche

1. Verfahren zur Abtrennung von Aminosäuren und/oder Aminosulfonsäuren von aus ihnen hergestellten Di-oder Oligopeptiden aus Lösungen
dadurch gekennzeichnet, daß man die Lösungen bei einem geeigneten pH-Wert mit einem Zeolith gegebenenfalls in mehreren Schritten in Kontakt bringt,und die in Lösung verbleibenden Produkte von den am Zeolith adsorbierten Aminosäuren und/oder Aminosulfonsäuren abtrennt.

2. Verfahren gemäß Anspruch 1,
dadurch gekennzeichnet, daß es sich bei den Di- oder Oligopeptiden um Verbindungen handelt, die ebenso wie die als Edukte eingesetzten Aminosäuren und/oder Aminosulfonsäuren sowohl Amino- als auch Säuregruppen enthalten.

3. Verfahren gemäß den Ansprüchen 1 + 2
dadurch gekennzeichnet, daß es sich bei den Di- oder Oligopeptiden um Verbindungen handelt, in denen mindestens zwei Aminosäuren und/oder Aminosulfonsäuren über eine Amidbindung verknüpft sind.

4. Verfahren gemäß den Ansprüchen 1 bis 3,
dadurch gekennzeichnet, daß man einen Zeolith der Typen TA, FAU, MOR oder MSI einsetzt.

5. Verfahren gemäß den Ansprüchen 1 bis 4,
dadurch gekennzeichnet, daß man die Adsorption der Aminosäure(n) und /oder Aminosulfonsäure(n) bei einem pH-Wert <JP (Isoelektrischer Punkt) durchführt.

6. Verfahren gemäß den Ansprüchen 1 bis 4,
dadurch gekennzeichnet, daß man bei Aminosäuren und/oder Aminosulfonsäuren mit unterschiedlichen JP-Werten,die in der aufzutrennenden Lösung nebeneinander vorliegen, gegebenenfalls zwei Adsorptionsschritte mit jeweils an den JP-Wert angepaßten pH-Wert durchführt.

7. Verfahren gemäß den Ansprüchen 1 bis 6
dadurch gekennzeichnet, daß man die an die Zeolithe adsorbierten Aminosäure(n) und/oder Aminosulfonsäure(n) durch Einstellen eines pH-Wertes >JP desorbiert.

8. Verfahren gemäß den Ansprüchen 1 bis 7
dadurch gekennzeichnet, daß die Amino- und Säuregruppen der Aminosäure und/oder Aminosulfonsäuren durch eine C₁-C₄-Alkylengruppe verbunden sind.

9. Verfahren gemäß den Ansprüchen 1 bis 8,
dadurch gekennzeichnet, daß die Di- oder Oligopeptide mindestens eine Aminosäure enthalten,die eine weitere funktionelle Gruppe besitzt.

10. Verfahren gemäß den Ansprüchen 1 bis 7,
dadurch gekennzeichnet, daß die Di- oder Oligopeptide mindestens eine Aminosäure mit einer hydrophoben Seitenkette enthalten.

11. Verfahren gemäß den Ansprüchen 1 bis 7,
dadurch gekennzeichnet, daß die Di- oder Oligopeptide mindestens eine Aminosäure aufweisen, bei der die Aminogruppe als primäres, sekundäres, oder tertiäres Amin vorliegt.

12. Verfahren gemäß den Ansprüchen 1 bis 7
dadurch gekennzeichnet, daß die Di- oder Oligopeptide Aminosäuren enthalten, deren Aminogruppe in der α- und/oder β- und/oder γ-Stellung vorliegt.

13. Verfahren gemäß den Ansprüchen 1 bis 11,
dadurch gekennzeichnet, daß man es batch-weise, halbkontinuierlich oder kontinuierlich durchführt.

## Claims

1. Process for the separation, from solutions, of amino acids and/or aminosulfonic acids from di- or oligopeptides prepared from them, characterised in that the solutions, at a suitable pH value, are brought into contact, optionally in several steps, with a zeolite and the products remaining in solution are separated from the amino acids and/or aminosulfonic acids adsorbed onto the zeolite.

2. Process according to claim 1,
characterised in that the di- or oligopeptides are compounds which, like the amino acids- and/or aminosulfonic acids used as educts, contain both amino groups and acid groups.

3. Process according to claims 1 and 2,
characterised in that the di- or oligopeptides are compounds in which at least two amino acids and/or aminosulfonic acids are bonded together by an amide linkage.

4. Process according to claims 1 to 3,
characterised in that a zeolite of the types TA, FAU, MOR or MSI is used.

5. Process according to claims 1 to 4,
characterised in that the adsorption of the amino acid(s) and/or aminosulfonic acid(s) is carried out at a pH value < IP (isoelectric point).

6. Process according to claims 1 to 4,
characterised in that where amino acids and/or aminosulfonic acids having different IP values are simultaneously present in the solution to be separated, optionally two adsorption steps are carried out, each with a pH value adjusted to the IP value.

7. Process according to claims 1 to 6,
characterised in that the amino acid(s) and/or aminosulfonic acid(s) adsorbed onto the zeolites is (are) desorbed by adjusting a pH value to > IP.

8. Process according to claims 1 to 7,
characterised in that the amino groups and acid groups of the amino acid and/or aminosulfonic acids are bonded together by a C₁-C₄-alkylene group.

9. Process according to claims 1 to 8,
characterised in that the di- or oligopeptides contain at least one amino acid which possesses an additional functional group.

10. Process according to claims 1 to 7,
characterised in that the di- or oligopeptides contain at least one amino acid having a hydrophobic side chain.

11. Process according to claims 1 to 7,
characterised in that the di- or oligopeptides contain at least one amino acid in which the amino group is present as primary, secondary or tertiary amine.

12. Process according to claims 1 to 7,
characterised in that the di- or oligopeptides contain amino acids the amino groups of which are in the α- and/or β- and/or γ-position.

13. Process according to claims 1 to 11,
characterised in that it is carried out *batchwise,* semi-continuously or continuously.

## Revendications

1. Procédé pour la séparation d'aminoacides et/ou d'acides aminosulfoniques de di- ou d'oligopeptides préparés à partir de ceux-ci, hors de solutions, caractérisé en ce qu'on amène les solutions à une valeur de pH appropriée en contact avec une zéolithe le cas échéant en plusieurs étapes et on sépare les produits restant en solution des aminoacides et/ou des acides aminosulfoniques adsorbés sur la zéolithe.

2. Procédé selon la revendication 1, caractérisé en ce que, en ce qui concerne les di- ou les oligopeptides, il s'agit de composés qui contiennent, au même titre que les aminoacides et/ou les acides aminosulfoniques mis en oeuvre à titre d'éduits, aussi bien des groupes amino que des groupes acides.

3. Procédé selon les revendications 1 et 2, caractérisé en ce que, en ce qui concerne les di- ou les oligopeptides, il s'agit de composés dans lesquels sont liés au moins deux aminoacides et/ou deux acides aminosulfoniques via une liaison amide.

4. Procédé selon les revendications 1 à 3, caractérisé en ce qu'on met en oeuvre une zéolithe des types TA, FAU, MOR ou MSI.

5. Procédé selon les revendications 1 à 4, caractérisé en ce qu'on effectue l'adsorption de l'aminoacide ou des aminoacides et/ou de l'acide aminosulfonique ou des acides aminosulfoniques à une valeur de pH < PI (point isoélectrique).

6. Procédé selon les revendications 1 à 4, caractérisé en ce que, dans le cas d'aminoacides et/ou d'acides aminosulfoniques possédant des valeurs PI ifférentes, qui sont présents conjointement dans la solution à séparer, on met en oeuvre le cas échéant deux étapes d'adsorption avec une valeur de pH respectivement adaptée à la valeur du PI.

7. Procédé selon les revendications 1 à 6, caractérisé en ce qu'on soumet à une désorption l'aminoacide ou les aminoacides et/ou l'acide aminosulfonique ou les acides aminosulfoniques adsorbés sur les zéolithes, en réglant une valeur de pH > PI.

8. Procédé selon les revendications 1 à 7, caractérisé en ce que les groupes amino et les groupes acides des aminoacides et/ou des acides aminosulfoniques sont liés via un groupe alkylène en C₁-C₄.

9. Procédé selon les revendications 1 à 8, caractérisé en ce que les di- ou les oligopeptides contiennent au moins un aminoacide qui possède un groupe fonctionnel supplémentaire.

10. Procédé selon les revendications 1 à 7, caractérisé en ce que les di- ou les oligopeptides contiennent au moins un aminoacide possédant une chaîne latérale hydrophobe.

11. Procédé selon les revendications 1 à 7, caractérisé en ce que les di- ou les oligopeptides présentent au moins un aminoacide dans lequel le groupe amino est présent sous forme d'une amine primaire, d'une amine secondaire ou d'une amine tertiaire.

12. Procédé selon les revendications 1 à 7, caractérisé en ce que les di- ou les oligopeptides contiennent des aminoacides dont le groupe amino est présent en position α et/ou en position β et/ou en position γ.

13. Procédé selon les revendications 1 à 11, caractérisé en ce qu'on l'effectue en discontinu, en semi-continu ou en continu.
